# EUROPEAN PATENT APPLICATION

(11) **EP 0 716 146 A2**
(43) Date of publication of application: **12.06.1996**
(21) Application number: 95300661.6
(22) Date of filing: 02.02.1995
(51) Int. Cl.: C12N 15/12, C07K 14/705, C07K 16/28

(54) **A novel stem cell marker protein, antibodies thereto, compositions thereof and nucleotide sequences encoding said protein**

(30) Priority: 06.12.1994 US 352323
(71) Applicant: SYSTEMIX, INC., Palo Alto, California 94304 (US)
(72) Inventor: Yang, Zhi, Menlo Park, California 94025 (US); Uchida, Nobuko, Palo Alto, California 94301 (US); Gearing, David, Mountain View, California 94040 (US)
(74) Representative: Goldin, Douglas Michael

(57) **Abstract**

Nucleotide sequences encoding a novel stem cell marker protein, the amino acid sequence of the protein, methods for expressing and purifying the protein, and antibodies against the protein, are provided.

## Description

### INTRODUCTION

### Technical Field

The field of this invention is the isolation of nucleotide sequences encoding a novel stem cell marker protein.

### Background

All publications cited herein are hereby incorporated herein by reference in their entirety.

Mammalian hematopoietic (blood) cells provide a diverse range of physiological activities. Blood cells are divided into lymphoid, myeloid and erythroid lineages. The lymphoid lineages, comprising B cells and T cells, provide for the production of antibodies, regulation of the cellular immune system, detection of foreign agents in the blood, detection of cells foreign to the host, and the like. The myeloid lineage, which includes monocytes, granulocytes and megakaryocytes, as well as other cells, monitors for the presence of foreign bodies, provides protection against neoplastic cells, scavenges foreign materials, produces platelets, and the like. The erythroid lineage provides the red blood cells, which act as oxygen carriers.

Despite the diversity of the natures, morphologies, characteristics and functions of blood cells, it is presently believed that all blood cells are derived from a single progenitor population, termed "stem cells." Stem cells are capable of self-regeneration and may become lineage committed progenitors that are dedicated to differentiation and expansion into a specific lineage.

A highly purified population of stem cells is necessary for a variety of *in vitro* experiments and *in vivo* indications. For instance, stem cells find use: (1) in regenerating the hematopoietic system of a host deficient in stem cells; (2) in treating a diseased host by removal of bone marrow, isolation of stem cells, treatment of the host with drugs or irradiation, and re-engraftment of stem cells; (3) in developing various hematopoietic cell lineages; (4) in detecting and evaluating growth factors relevant to stem cell self-regeneration; (5) in detecting and evaluating growth factors associated with the early steps of commitment of a stem cell to a particular lineage; (6) as a target for gene therapy to endow blood cells with useful properties; (7) in surgeries involving hematopoietic engraftment including, but not limited to, hematopoietic engraftment in cancer patients and transplantation of other organs in association with hematopoietic engraftment; and (8) in the treatment of lymphomas and leukemias, as well as other neoplastic conditions. Thus, there have been world-wide efforts toward isolating the human hematopoietic stem cell in substantially pure or pure form.

Hematopoietic cells are identifiable by the presence of a variety of cell surface "markers." Such markers may be either specific to a particular lineage or progenitor cell or be present on more than one cell type. Typically, enrichment for stem cells relies on positive selection for cells bearing surface markers present on stem cells and/or negative selection to remove cells bearing surface markers absent on stem cells. For example, stem cells are phenotypically LIN⁻ (which generally means that they lack markers associated with T cells (such as CD2, 3, 4 and 8), B cells (such as CD10, 19 and 20), myeloid cells (such as CD14, 15 and 16), natural killer ("NK") cells (such as CD2, 16 and 56) and red blood cells (such as glycophorin A). The presence or absence of protein markers is identified by the binding or lack thereof of antibodies specific to the markers.

Unfortunately, to date, no marker specific to stem cells has been identified, nor is it clear how many of the markers associated with differentiated cells are also present on stem cells. For example, CD34, which was originally identified as stem cell specific, is also found on a significant number of lineage committed cells: 80-90% of the CD34⁺ population is marked by other lineage-specific and non-specific markers. U.S. Pat. No. 4,714,680 describes a composition comprising CD34⁺ cells. As a result of the above uncertainties, the isolation of a pure composition of human stem cells has remained elusive. The identification of new markers associated with stem cells will greatly aid the effort to achieve this goal. Characterization and isolation of human hematopoietic stem cells are reported in: Baum et al. (1991) Proc. Natl. Acad. Sci. USA; and Tsukamoto et al. U.S. Patent No. 5,061,620.

Recently, the mouse stem cell was obtained in at least highly concentrated, if not a purified form, where fewer than about 30 cells obtained from bone marrow were able to reconstitute all of the lineages of the hematopoietic system of a lethally irradiated mouse. Each assayed cell is multipotent for all blood lineages, while self-renewal is variable amongst these cells. Spangrude et al. (1988) Science 241:58-62; Smith et al. (1991) Proc. Natl. Acad. Sci. 88:2788-2792; Uchida Ph.D. Thesis. Stanford U. (1992); and see also, EPA 89.304651.6 and the references cited therein which describe the isolation of mouse stem cells.

### SUMMARY OF THE INVENTION

Nucleotide sequences encoding a novel marker protein associated with stem cells are provided. This protein is designated HCA for human hematopoietic cell antigen. Two nucleotide sequences, HCASeq.1 and HCASeq.2, which are likely to be splice variants of each other, and vectors containing the sequences are provided. Also provided are the amino acid sequences of the HCA proteins, HCAPro.1 and HCAPro.2, encoded by these nucleotide sequences, methods for expressing and purifying these proteins, compositions of pure protein, and antibodies that bind with high specificity to one or more epitopes on these proteins. Patent publication PCT/US94/08574 filed July 29, 1994, provides methods for purifying human stem cells using a monoclonal antibody (F84.1 mAb) that recognizes HCA.

### BRIEF DESCRIPTION OF THE DRAWINGS

Figure 1 depicts an outline of the RT-PCR cloning approach used in the isolation of nucleotide sequences encoding HCA.

Figure 2 depicts the structures of the three genomic clones that were used to deduce one of the two identified full-length HCA nucleotide sequences.

Figures 3A and 3B depict both identified full-length nucleotide sequences of HCA, HCASeq.1 and HCASeq.2, respectively.

Figures 4A and 4B depict the predicted amino acid sequences of the proteins encoded by the nucleotide sequences in Figures 3A and 3B respectively (HCAPro. 1 and HCAPro. 2, respectfully).

Figure 5 depicts a Northern analysis of various human tissues using radiolabeled HCA1.4 as a probe.

Figure 6 depicts the expression construct pcD5neg-1/HCA1.4.

Figure 7 depicts the Biosensor assay results that demonstrate that F84.1 mAb recognizes a fusion protein of HCA and Fc.

### DESCRIPTION OF THE SPECIFIC EMBODIMENTS

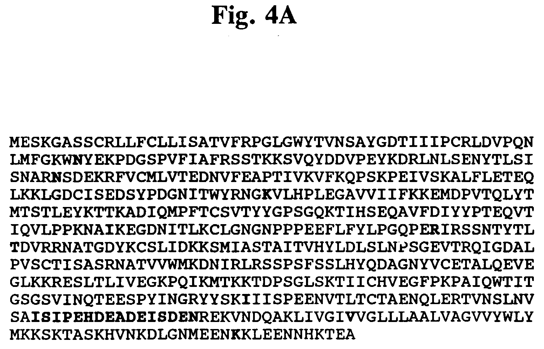

As used herein, HCA refers to the human protein that carries the epitope recognized by the monoclonal antibody designated F84.1 mAb and to all variants of this protein, including, but not limited to, variants resulting from alternative processing of the nucleotide sequences encoding this protein and fusion proteins as depicted in Figures 4A-B. As used herein, αHCA means F84.1 mAb, and any monoclonal antibody or polyclonal antibody, that binds to HCA. This also includes any antibody having the same antigenic specificity as F84.1 mAb.

HCA was recognized by a monoclonal antibody, designated F84.1 mAb, that recognized a rat protein designated F84.1. F84.1 is expressed in the rat nervous system with a distribution similar to that of a chicken protein called BEN/DM-GRASP/SC1, which is a member of the immunoglobulin superfamily. Pourquié et al. (1992) Dev. Bio. 89:5261-5265. Antibodies to BEN/DM-GRASP/SC1 also recognized human embryonic motor neurons. Pourquié et al. "Expression des proteines d'adherence BEN/SC1/DM-GRASP et Tag-1 pendant l'axogenese des motoneurones humans" (1994). A monoclonal antibody that recognized BEN/DM-GRASP/SC1 also stained avian hematopoietic cells, including differentiating T cells, CD4⁺CD8⁺ T cells and myeloid and erythroid progenitors, but not B cells. Corbel et al. (1992) Exp. Cell Res. 203:91-99. The similar nervous system distributions, of F84.1 and BEN/DM-GRASP/SC1 led to the hypothesis that F84.1 is a rat homolog of BEN/DM-GRASP/SC1. N-terminal sequencing of F84.1 supported this hypothesis. Prince et al. (1992) Devel. Br. Res. 68:193-201. Similarly, a protein designated KG-CAM was proposed to be another rat homolog of BEN/DM-GRASP/SC1. Peduzzi et al. (1994) Brain Res. 640:296-307. The recognition of HCA, which is expressed on human hematopoietic stem cells, by F84.1 mAb led to the hypothesis that HCA is a human homolog of the rat proteins F84.1 and KG-CAM and the chicken protein BEN/DM-GRASP/SC1.

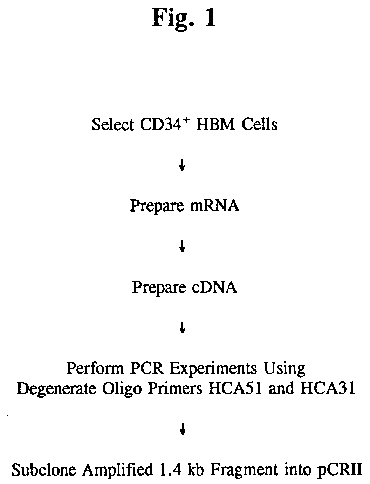

In one embodiment, the present invention provides nucleotide sequences encoding HCA. Two different nucleotide sequences, HCASeq.1 and HCASeq.2, which are likely to be splice variants of each other, are provided. Nucleotide sequences encoding HCA were isolated from a bone marrow CD34⁺ cDNA library by RT-PCR cloning (Figure 1) using the degenerate primers shown in Example 1.

In another embodiment, nucleotide sequences encoding HCA variants including, e.g., other alternatively processed sequences or sequences encoding HCA fusion and deletion proteins, are provided. Alternatively processed nucleotide sequence variants are defined as nucleotide sequences corresponding to mRNAs that differ in sequence from one another but are derived from the same genomic region, for example, mRNAs that result from: 1) the use of alternative promoters; 2) the use of alternative polyadenylation sites; or 3) the use of alternative splice sites.

As used herein, nucleotides encompasses RNA, cDNA, genomic DNA, synthetic forms, mixed polymers, both sense and antisense strands and nucleotides of less than the full length cDNA sequences. Preferably, the nucleotides are at least 200 bases in length. More preferably, the nucleotides are 100 bases in length. Most preferably, the nucleotides are 50 bases in length. The nucleotides will usually comprise sequences corresponding to at least about 12 to 15 nucleotides (or base pairs), more usually at least about 21 nucleotides, and most preferably at least about 35 nucleotides, the length depending on the desired use. One or more introns may also be present. In all instances, the nucleotides are specific to the human sequence and are not the same as, or homologous to, sequences from other sources.

The polynucleotides may be chemically or biochemically modified or contain non-natural or derivatized nucleotide bases. The nucleotides may be complementary to the mRNA encoding at least a fragment of the HCA proteins and other nucleotides which can bind to either the DNA or mRNA encoding the HCA proteins. These complementary nucleotides include, but are not limited to, nucleotides capable of forming triple helices and antisense nucleotides. The complementary nucleotides may be expressed endogenously by a vector or may be added exogenously by methods known in the art of oligonucleotide therapy. Reed at al. (1990) Cancer Res. 50:6565-6570. Recombinant polynucleotides comprising sequences otherwise not naturally occurring are also provided by this invention, as are alterations of wild type nucleotide sequences, including but not limited to, those due to deletion, insertion, substitution of one or more nucleotides or by fusion to other polynucleotide sequences.

Methods for constructing appropriate cDNA and genomic libraries, for screening libraries for sequences of interest, for preparing suitable probes or primers (e.g., PCR primers), for polynucleotide purification, amplification and subcloning, and for host cell transformation and other techniques of recombinant DNA technology appropriate to the practice of the present invention are provided, inter alia, in Sambrook et al. (1989); Ausubel et al. (1987 and periodic updates); and PCR Protocols: A Guide to Methods and Applications, Innis et al., eds., Academic Press: San Diego (1990). Reagents useful in applying such techniques, such as restriction enzymes, expression vectors, labels, etc. are known in the art and commercially available from such vendors as New England BioLabs, Boehringer Mannheim, Amersham, Promega Biotec, U. S. Biochemicals, New England Nuclear, and a number of other commercial sources.

In another embodiment, nucleotides homologous to sequences encoding HCA are provided. A nucleotide or fragment thereof is "substantially homologous" (or "substantially similar") to another if, when optimally aligned (with appropriate nucleotide insertions or deletions) with another nucleotide (or its complementary strand), there is nucleotide sequence identity in at least about 60% of the nucleotide bases, usually at least about 70%, more usually at least about 80%, preferably at least about 90%, and more preferably at least about 95 to 98% of the nucleotide bases. Also included are those nucleotides which encode HCA but which have nucleotide substitutions that accommodate the degeneracy of the genetic code.

Alternatively, substantial homology or (similarity) exists when a nucleotide or fragment thereof will hybridize to another nucleotide (or a complementary strand thereof) under selective hybridization conditions. Selectivity of hybridization exists under hybridization conditions which allow one to distinguish the target nucleotide of interest from other polynucleotides. Typically, selective hybridization will occur when there is at least about 55% similarity over a stretch of at least about 14 nucleotides, preferably at least about 65%, more preferably at least about 75%, and most preferably at least about 90%. See, Kanehisa (1984) Nuc. Acids Res. 12:203-213. The length of homology comparison, as described, may be over longer stretches, and in certain embodiments will often be over a stretch of at least about 17 to 20 nucleotides, and preferably at least about 36 or more nucleotides.

The hybridization of nucleotides is affected by such conditions as salt concentration, temperature, or organic solvents, in addition to the base composition, length of the complementary strands, and the number of nucleotide base mismatches between the hybridizing polynucleotides, as will be readily appreciated by those skilled in the art. Stringent temperature conditions will generally include temperatures in excess of 30°C, typically in excess of 37°C, and preferably in excess of 45°C. Stringent salt conditions will ordinarily be less than 1 M, typically less than 500 mM, and preferably less than 200 mM. However, the combination of parameters is much more important than the measure of any single parameter. Wetmur and Davidson (1968) J. Mol. Biol. 31:349-370.

An "isolated" or "substantially pure" nucleotide is a nucleotide which is substantially separated from other nucleotides which naturally accompany a native nucleotide sequence. The term embraces a nucleotide sequence which has been removed from its naturally occurring environment, and includes recombinant or cloned DNA isolates and chemically synthesized analogues or analogues biologically synthesized by heterologous systems.

A nucleotide is said to "encode" a polypeptide if, in its native state or when manipulated by methods well known to those skilled in the art, it can be transcribed and/or translated to produce the polypeptide or a fragment thereof. The anti-sense strand of such a polynucleotide is also said to encode the sequence.

A polynucleotide sequence is operably linked when it is placed into a functional relationship with another polynucleotide sequence. For instance, a promoter is operably linked to a coding sequence if the promoter affects its transcription or expression. Generally, operably linked means that the sequences being linked are contiguous and, where necessary to join two protein coding regions, contiguous and in reading frame. However, it is well known that certain genetic elements, such as enhancers, may be operably linked even at a distance, i.e., even if not contiguous.

The term "recombinant" polynucleotide refers to a polynucleotide which is made by the combination of two otherwise separated segments of sequence accomplished by the artificial manipulation of isolated segments of polynucleotides by genetic engineering techniques or by chemical synthesis. In so doing one may join together polynucleotide segments of desired functions to generate a desired combination of functions.

Polynucleotide probes include an isolated polynucleotide attached to a label or reporter molecule and may be used to identify and isolate other sequences. Probes comprising synthetic oligonucleotides or other polynucleotides may be derived from naturally occurring or recombinant single or double stranded nucleic acids or may be chemically synthesized. Polynucleotide probes may be labelled by any of the methods known in the art, e.g., random hexamer labeling, nick translation, or the Klenow fill-in reaction.

Large amounts of the polynucleotides may be produced by replication in a suitable host cell. Natural or synthetic DNA fragments encoding protein(s) or a fragment thereof will be incorporated into recombinant polynucleotide constructs, typically DNA constructs, capable of introduction into and replication in a prokaryotic or eukaryotic cell. Usually the construct will be suitable for replication in a unicellular host, such as yeast or bacteria, but a multicellular eukaryotic host may also be appropriate, with or without integration within the genome of the host cells. Commonly used prokaryotic hosts include strains of *Escherichia coli,* although other prokaryotes, such as *Bacillus subtilis* or *Pseudomonas* may also be used. Mammalian or other eukaryotic host cells include yeast, filamentous fungi, plant, insect, amphibian or avian species. Such factors as ease of manipulation, ability to appropriately glycosylate expressed protein(s), degree and control of protein expression, ease of purification of expressed protein(s) away from cellular contaminants or other factors may determine the choice of the host cell.

The polynucleotides may also be produced by chemical synthesis, e.g., by the phosphoramidite method described by Beaucage and Carruthers (1981) Tetra. Letts. 22:1859-1862 or the triester method according to Matteucci et al. (1981) J. Am. Chem. Soc. 103:3185, and may be performed on commercial automated oligonucleotide synthesizers. A double-stranded fragment may be obtained from the single stranded product of chemical synthesis either by synthesizing the complementary strand and annealing the strand together under appropriate conditions or by adding the complementary strand using DNA polymerase with an appropriate primer sequence.

In another embodiment, expression systems are provided for production of recombinant HCA proteins. Expression systems are defined as polynucleotides which, when transformed into an appropriate host cell, can express a protein(s). The polynucleotides possess a nucleotide sequence that is substantially similar to a natural protein-encoding polynucleotide or a fragment thereof.

Expression systems prepared for introduction into a prokaryotic or eukaryotic host will typically comprise a replication system recognized by the host, including the intended DNA fragment encoding the desired polypeptide, and will preferably also include transcription and translational initiation regulatory sequences operably linked to the polypeptide encoding segment. Expression systems (expression vectors) may include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. DNA encoding signal peptides may also be included where appropriate from secreted polypeptides of the same or related species, which allow the protein to cross and/or lodge in cell membranes or be secreted from the cell.

The selection of an appropriate promoter and other necessary vector sequences will be selected so as to be functional in the host. Examples of workable combinations of cell lines and expression vectors are described in Sambrook et al., 1989; Ausubel et al., 1987; and Metzger et al. (1988) Nature 334:31-36. Many useful vectors for expression in bacteria, yeast, mammalian, insect, plant or other cells are well known in the art and may be obtained from such vendors as Stratagene, New England Biolabs, Promega Biotech, and others. In addition, the construct may be joined to an amplifiable gene (e.g., DHFR) so that multiple copies of the gene may be made. For appropriate enhancer and other expression control sequences see also Enhancers and Eukaryotic Gene Expression, Cold Spring Harbor Press, N.Y. (1983). While such expression vectors may replicate autonomously, they may less preferably replicate by being inserted into the genome of the host cell.

Expression and cloning vectors will likely contain a selectable marker. Selectable markers are produced by genes encoding a protein necessary for the survival or growth of a host cell transformed with the vector. Such a selectable gene may also be carried on another polynucleotide sequence co-introduced with the expression vector into the host cell. Only those host cells into which the selectable gene has been introduced will survive and/or grow under selective conditions. Typical selection genes encode protein(s) that (a) confer resistance to antibiotics or other toxic substances, e.g. ampicillin, neomycin, methotrexate, etc.; (b) complement auxotrophic deficiencies, or (c) supply critical nutrients not available from complex media. The choice of the proper selectable marker will depend on the host cell, and appropriate markers for different hosts are known in the art.

The vectors containing the polynucleotides of interest can be introduced into the host cell by any of a number of appropriate means, including electroporation; transfection employing calcium chloride, rubidium chloride, calcium phosphate, DEAE-dextran, or other substances; microprojectile bombardment; lipofection; infection (where the vector is an infectious agent, such as a retroviral genome). The choice of such means will often depend on the host cell.

Large quantities of the polynucleotides and polypeptides of the present invention may be prepared by transforming suitable prokaryotic or eukaryotic host cells with protein(s)-encoding polynucleotides of the present invention in compatible vectors or other expression vehicles and culturing such transformed host cells under conditions suitable to attain expression of the protein(s)-encoding gene. The protein(s) may then be recovered from the host cell and purified using standard techniques. The endogenous gene may also be used to produce protein. The native expression may be suitable for small amounts of protein and expression levels can be increased by upstream activation of expression. Methods of upstream activation are known and include insertion of activation sequences upstream of the endogenous HCA gene by homologous recombination.

In another embodiment, methods of identifying non-hematopoietic stem cells, particularly those of the nervous system, are provided. HCA has now been found to be one of a group of markers expressed in both neural and hematopoietic systems including Thy-1, c-kit and LIF-Receptor. HCA expression during neural development is highest in regions having large numbers of primitive cells. Thus in one embodiment of the invention, identification of non-hematopoietic stem cells is provided by monitoring the expression of HCA. The methods of identifying the cells include any known in the arts of mRNA and protein detection. Suitable methods of isolating and manipulating neural stem cells are known in the art and described for instance in Reynolds and Weiss (1992) Science 255:1707; Hall et al. (1989) Development 106:619; Potten et al. (1990) Crypt. Development 110:100; Dupin et al. (1990) Proc. Natl. Acad. Sci. USA 87:1119 and PCT publication no. WO 94/16718. In another embodiment of the invention, the purified HCA proteins are provided. The predicted amino acid sequences of the HCA protein are provided. The proteins can be isolated from recombinant expression systems, native sources or manufactured synthetically. Preferably, the proteins are at least partially purified from other cellular constituents. Preferably, the proteins are at least 50% pure. More preferably, the proteins are 50-75% pure. More highly purified proteins may also be obtained and are encompassed by the present invention.

Methods are provided for expressing HCA proteins or HCA fusion proteins and purifying the expressed proteins. Purification or isolation of HCA proteins expressed either by the recombinant DNA or from endogenous genes can be accomplished by any method known in the art. For antibody production, the proteins are preferably highly purified, usually about 99% pure, and free of pyrogens and other contaminants.

Suitable methods of protein purification are known in the art and include, but are not limited to, affinity chromatography, immunoaffinity chromatography, size exclusion chromatography, HPLC and FPLC. Any purification scheme that does not result in substantial degradation of the protein is suitable for use in the present invention.

The invention encompasses fragments of HCA proteins which contain at least one antigenic determinant. As used herein, "HCA protein" encompasses the full length proteins and any such fragments.

The invention encompasses functionally equivalent variants of HCA proteins which do not significantly affect their properties and variants which retain the same overall amino acid sequence but which have enhanced or decreased activity. For instance, conservative substitutions of amino acid residues, one or a few amino acid deletions or additions, and substitution of amino acid residues by amino acid analogs are within the scope of the invention. Any conservative amino acid substitution which does not significantly affect the properties of HCA proteins is encompassed by the present invention. Amino acid residues which can be conservatively substituted for one another include, but are not limited to: glycine/alanine; valine/isoleucine/ leucine; asparagine/glutamine; aspartic acid/glutamic acid; serine/threonine; lysine/arginine; and phenylalanine/tyrosine. Any conservative amino acid substitution which does not significantly affect the properties of HCA proteins is encompassed by the present invention.

In another embodiment, polyclonal and/or monoclonal antibodies, or functional portions thereof, capable of specifically binding to HCA and any and all variants or fragments thereof are provided. The term antibody is used to refer both to a homogeneous molecular entity, or a mixture such as a serum product made up of a plurality of different molecular entities. Monoclonal or polyclonal antibodies reacting with the HCA protein(s) may be made by methods known in the art. See, e.g., Harlow and Lane, (1988) Antibodies: A Laboratory Manual, CSH Laboratories; Goding (1986) Monoclonal Antibodies: Principles and Practice, 2d ed, Academic Press, New York; and Ausubel et al. (1987). Also, recombinant immunoglobulins may be produced by method known in the art, including but not limited to, the methods described in U.S. Patent No. 4,816,567. Monoclonal antibodies with affinities of 10⁸ M⁻¹ preferably 10⁹ to 10¹⁰ or more are preferred although for many applications, lower affinity may be preferred for competitive release systems.

Antibodies specific for the protein may be useful in purifying stem cells from both hematopoietic and other sources. Such antibodies, whether monoclonal or polyclonal, can be made by methods known in the art utilizing purified HCA as the immunogen. Frequently, the polypeptides and antibodies will be labeled by joining, either covalently or noncovalently, a substance which provides a detectable signal. Suitable labels include, but are not limited to, radionuclides, enzymes, substrates, cofactors, inhibitors, fluorescent agents, chemiluminescent agents, magnetic particles and the like. United States Patents describing the use of such labels include but are not limited to, Nos. 3,817,837; 3,850,752; 3,939,350; 3,996,345; 4,277,437; 4,275,149; and 4,366,241.

The following examples are offered to illustrate but not limit the invention.

### Example 1

### Isolation of the nucleotide sequence encoding HCA

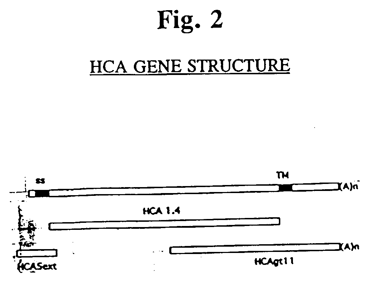

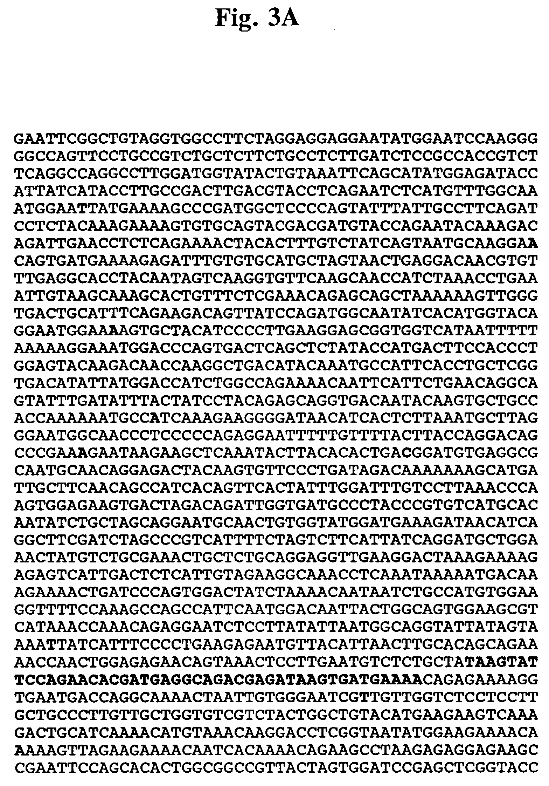

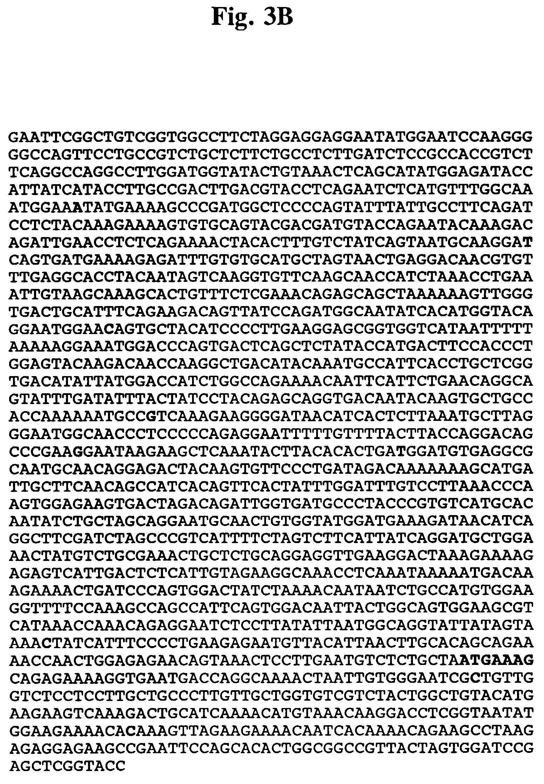

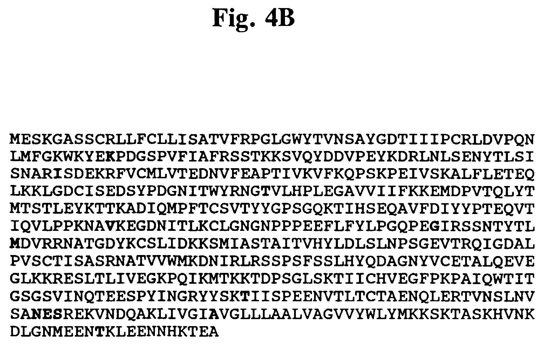

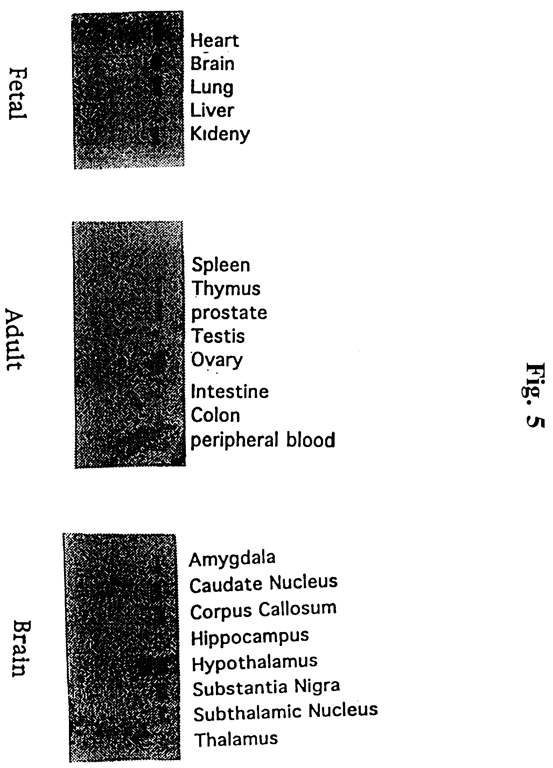

The nucleotide sequence encoding HCA was isolated by reverse transcriptase PCR (RT-PCR) Cloning (Fig. 1). Degenerate 5' and 3' oligonucleotide primers were made. These primers (HCA51 and HCA31) had the following sequences: 
          T    T                                                    T
HCA51:  5'TA(TC)AC(A/G)GT(A/G)AA(T/C)TCAGCATA(T/C)GGAGA(T/C)AC(A/G)AT 3'
          C    C                                                    C
 HCA31: 5' TT(T/C)TC(T/C)TCTAG(T/C)TT(T/C)TT(G/A)TT(T/C)TC(T/C)TC 3' HCA51 and HCA31 were used to isolate the nucleotide sequence encoding the extracellular domain of HCA from CD34-selected human bone marrow (HBM) cells using RT-PCR. A 1.4 kb fragment was dominantly amplified (HCA1.4). This fragment was purified, cloned into the pCRII vector, and sequenced to confirm that it encoded a peptide similar in sequence to the extracellular domains of KG-CAM and BEN/DM-GRASP/SC1. Two full-length HCA sequences were identified. The first sequence was obtained from three overlapping clones (Fig. 2). HCAgt11 was isolated from a λgt11 human brain cDNA library by was isolated from a λgt11 human brain cDNA library by hybridization of radiolabeled HCA1.4 to sequences in the library. HCA5ext was isolated from HBM cDNA using 5' RACE. The second nucleotide sequence was obtained from human CD34⁺ cell cDNA using RT-PCR. Both full-length nucleotide sequences (Fig. 3A and 3B) and the predicted amino acid sequences of the corresponding proteins (Fig. 4A and 4B) are provided. The two nucleotide sequences appear to differ from one another as a result of the use of alternative splice sites near the 3' ends of the sequences with the result that each sequence has nucleotides specific to it. These nucleotides are in bold. The proteins encoded by these two nucleotide sequences also differ from one another near their carboxy termini, with each protein sequence containing amino acids that are specific to it. These amino acids are also in bold. In addition to the above differences, there are, throughout the protein, single nucleotide differences that result in amino acid changes. These differences are also in bold. The origins of the single nucleotide changes are unknown. HCA was shown by Northern blot analysis to be expressed in many human tissues both early in development and in adults (Fig. 5).

### Example 2

### Expression of a HCA/Fc fusion protein and demonstration that F84.1 mAb recognizes this fusion protein

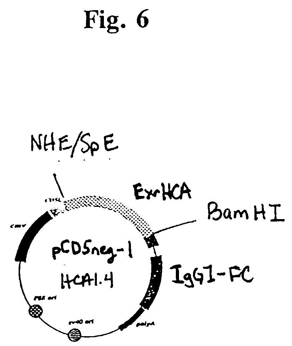

A fusion protein between the extracellular domain of HCA and Fc was produced and shown to be recognized by F84.1 mAb. To produce the fusion protein, that portion of the HCA nucleotide sequence encoding the extracellular domain of the protein was cloned into the pCD5neg-1 expression vector as follows. PCR reactions were carried out using HCA1.4 in pCRII as the template and a 5' primer that included a SpeI site and a 3' primer that included a BamHI site. The amplified DNA fragment was cut with SpeI and BamHI and cloned into the pCD5neg-1 vector. The resulting construct (pCD5neg-1/HCA1.4) is depicted in Figure 6. The construct was transfected into COS7 cells to produce a HCA-Fc fusion protein (HCAFc). Fusion proteins were purified from the COS7 supernatant over protein A.

That F84.1 mAb recognizes HCAFc was demonstrated using ELISA and Biosensor assays. For the ELISA assay, HCAFc was coated onto an Immulon 4 ELISA plate at 10 micrograms/milliliter in 50 millimolar carbonate/bicarbonate buffer, pH 9.6, overnight. Plates were blocked using 2% bovine serum albumin in 0.05% Tween-20, PBS, pH 7.2, for an hour at room temperature and then samples containing F84.1 mAb or relevant controls (IgG1) were added. Controls were run at 10 micrograms/milliliter while hybridoma supernatants were tested undiluted or as 1/5 dilutions.

Following incubation of the monoclonal antibodies for 1 hour at room temperature, the plates were washed using PBS/0.05% Tween-20, followed by incubation of the plates with alkaline phosphatase labelled goat anti-mouse Ig at 1/1000 dilution for 1 hour at room temperature. The plates were washed and developed using PNPP as the substrate. The reactions were stopped using 10 millimolar EDTA and read at 405 nm. The results demonstrated that F84.1 antibody recognizes HCAFc.

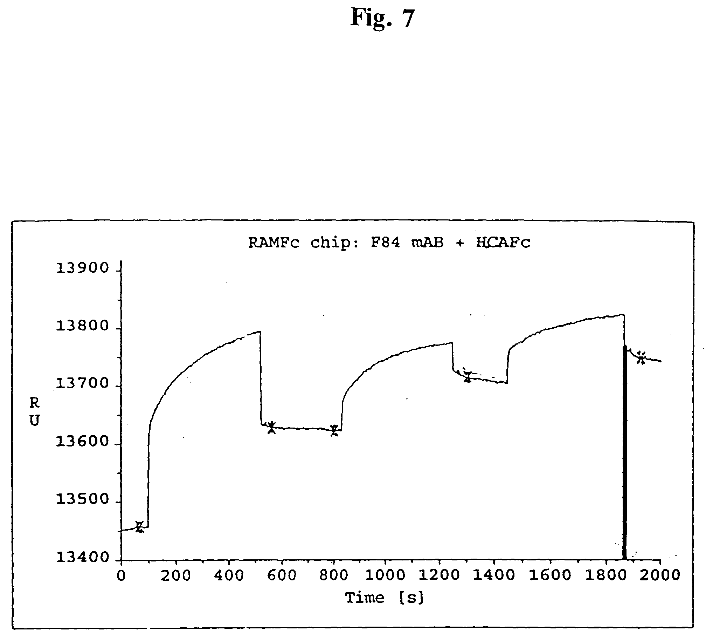

For the Biosensor assay, rabbit anti-mouse Fc was covalently coupled to a CM5 Biosensor chip at 50 micrograms/milliliter in NHS/EDC, followed by ethanolamine blocking according to the manufacturer's instructions (Pharmacia). F84.1 hybridoma supernatant was injected into the cell at 5 microliters/minute. Following this, purified HCA-Fc was injected at 5 microliters/minute. The difference in resonance units (RU) was determined and denotes the binding of F84.1 mAb to HCAFc (Fig. 7). The results of these assays demonstrate that F84.1 mAb recognizes HCAFc.

The compositions of pure protein obtained can be used to study the functions of the entire protein, or fragments thereof, using standard assays. The compositions of pure protein may also be used as antigens to produce antibodies against the entire or specific fragments of the protein.

Although the foregoing invention has been described in some detail by way of illustration and example for purposes of clarity and understanding, it will be apparent to those skilled in the art that certain changes and modifications may be practiced. Therefore, the descriptions and examples should not be construed as limiting the scope of the invention which is delineated by the appended claims.

## Claims

1. A composition comprising at least a portion of a nucleotide depicted in Figure 3A or 3B, or complements thereof.

2. A composition according to claim 1 wherein the nucleotide is at least 50 bases in length.

3. A composition according to claim 1 or 2 wherein the nucleotide is at least 100 bases in length.

4. The composition according to any one of claims 1 to 3 wherein the nucleotide is at least 200 bases in length.

5. A composition according to any one of claims 1 to 4 wherein the nucleotide encodes at least a fragment of a human HCA protein.

6. An expression vector comprising a nucleotide sequence according to claim 5 operably linked to a promoter compatible with a desired host cell.

7. A method of preparing human HCA protein or a fragment thereof which comprises expressing a vector according to claim 6 in a host cell and recovering the protein or fragment thereof.

8. A composition comprising an isolated protein depicted in Figure 4A or 4B, or fragments thereof.

9. A composition comprising an antibody generated in response to the protein according to claim 8.
